# EUROPEAN PATENT SPECIFICATION

(11) **EP 2 931 913 B1**
(45) Date of publication and mention of the grant of the patent: **17.10.2018**
(21) Application number: 13863073.6
(22) Date of filing: 11.12.2013
(51) Int. Cl.: C12N 15/10, C12P 19/34, C12Q 1/6844

(54) **PEG-MEDIATED ASSEMBLY OF NUCLEIC ACID MOLECULES**
PEG-VERMITTELTE ANORDNUNG AUS NUKLEINSÄUREMOLEKÜLEN
ASSEMBLAGE À MÉDIATION PAR PEG DE MOLÉCULES D'ACIDE NUCLÉIQUE

(30) Priority: 13.12.2012 US 201261736946 P
(43) Date of publication of application: 21.10.2015
(73) Proprietor: Synthetic Genomics, Inc., La Jolla, CA 92037 (US)
(72) Inventor: URANO, Jun, Irvine, CA 92620 (US); GIBSON, Daniel, G., Carlsbad, CA 92008 (US); CAIAZZA, Nicky, C., Rancho Santa Fe, CA 92067 (US); QI, Zhiqing, Charlotte, NC 28277-4180 (US)
(74) Representative: Cornish, Kristina Victoria Joy
(86) International application number: PCT/US2013/074471
(87) International publication number: WO 2014/093535

(56) References cited:
- WO-A1-2009/006598
- WO-A1-2014/028895
- WO-A2-2009/103027
- US-A- 5 928 905
- LEE, WH.: 'Gene Synthesis From Oligonucleotides Mixtures By Solid Phase PCR And Assembly PCR' A MICROFLUIDIC CHIP SYSTEM [DISSERTATION]. 2010, ANN ARBOR, MI, XP055255898
- YE, H. ET AL.: 'Experimental Analysis Of Fene Assembly With TopDown One-Step Real-Time Gene Synthesis.' NUCLEIC ACIDS RES. vol. 37, no. 7 : E5, April 2009, page 2, XP055038670
- STEMMER, WPC. ET AL.: 'Single-Step Assembly Of A Gene And Entire Plasmid From Large Numbers Of Oligodeoxyribonucleotides.' GENE vol. 164, no. 1, 15 October 1995, pages 49 - 53, XP004041916
- GRUNENWALD, H.: 'Optimization Of Polymerase Chain Reactions.' METHODS MOL. BIOL. vol. 226, 2003, pages 89 - 100, XP008179348
- FINNZYMES OY.: 'Phusion High-Fidelity DNA Polymerase [product datasheet].' June 2007, ESPOO, FINLAND, page 1, XP055255907

## Description

### Field of the Invention

The invention relates to the assembly of nucleic acid molecules. The invention will find application in diverse areas such as the construction of diverse synthetic metabolic pathways, automated DNA assembly, and robust engineering of large DNA fragments, among other areas.

### Background

The following description of the background of the invention is provided to aid in understanding the invention, but is not admitted to be, or to describe, prior art to the invention.

Synthetic gene construction finds application in many areas of molecular biology. DNA sequences can be assembled using various methods. These methods generally involve a two-step process of synthesis and amplification, where in a first step a set of overlapping oligonucleotides are synthesized using standard techniques for the synthesis of oligonucleotides, and assembled based on self-priming of the oligonucleotides through the homology between the overlapping areas. In a second step the assembled nucleic acid is subjected to PCR for amplification using an additional pair of primers to amplify the full-length gene product. Some available methods have relied on DNA polymerase to build increasingly longer DNA fragments during the assembly process.

Other nucleic acid assembly techniques have included the amplification primers in the original gene assembly mix. These methods have either been inefficient, have been able to assemble only smaller genes, or have been unable to assemble nucleic acids having challenging nucleotide content, such as being rich in AT or GC sequences.

Normally the assembly of a nucleic acid construct requires at least two steps: a first step for the pre-assembly of oligonucleotides, and a second step of amplification and assembly of the products of the pre-assembly in a separate PCR step
LEE, Woong Hee.: "Gene Synthesis From Oligonucleotides Mixtures By Solid Phase PCR And Assembly PCR in a Microfluidic Chip System" [DISSERTATION in The University of Michigan]., 2010, ANN ARBOR, MI discloses assembly PCR in which PEG8000 is used in a passivation step on the chip surface before the assembly PCR.

It would be advantageous to have a method for assembling nucleic acids or genes that could achieve the assembly and amplification of the desired nucleic acid or gene in a single step, and which could also synthesize nucleic acids and genes of larger size than has previously been available. It would also be advantageous to have a method that could perform the assembly in a single step.

### Summary

The present invention discloses methods for assembling a nucleic acid molecule in a single step from a set of overlapping oligonucleotides, as defined in claim 1. The method involves contacting a set of overlapping oligonucleotides with a DNA polymerase, a mixture of dNTPs, and a crowding agent to form an assembly mixture. The crowding agent is polyethylene glycol (PEG). The presence of the crowding agent facilitates the nucleic acid assembly process of the invention. The assembly mixture is then subjected to multiple cycles, each cycle comprising a denaturation phase, an annealing phase, and an extension phase, and the desired nucleic acid molecule is thereby assembled. In some embodiments one or more of the phases are time varied. The methods are performed in a single step.

The present invention provides methods for assembling a nucleic acid molecule in a single step from a set of overlapping oligonucleotides. The methods include (a) contacting a set of overlapping oligonucleotides with a DNA polymerase, a mixture of dNTPs, and polyethylene glycol to form an assembly mixture; (b) subjecting the assembly mixture to multiple cycles, each cycle comprising a denaturation phase, an annealing phase, and an extension phase, and (c) thereby assembling the nucleic acid molecule from a set of overlapping oligonucleotides in a single step.

In one embodiment the set of oligonucleotides contains end oligonucleotides and non-end oligonucleotides, and the end oligonucleotides are provided in the assembly mixture at a higher concentration than the non-end oligonucleotides. In some embodiments the at least one annealing phase occurs at a temperature of between 57 °C and 77 °C. The extension phase of each cycle can be increased in time relative to the extension phase of the previous cycle. The DNA polymerase can be a heat-stabile DNA polymerase, such as PHUSION® DNA polymerase (Finnzymes, Oy, FI). The set of oligonucleotides can be assembled into a gene. In some embodiments the polyethylene glycol is PEG 8000. The concentration of PEG can be 0.025% (w/v) or greater, or 0.375% (w/v) or greater. The annealing phase can occur at 67 °C, and the annealing and extension phases can be combined into a single phase. In various embodiments the nucleic acid molecule can be greater than 1 kb in length, or greater than 2 kb in length, or greater than 3 kb in length. The set of overlapping oligonucleotides can have at least 5 oligonucleotides, or at least 60 oligonucleotides, or at least 75 oligonucleotides.

In one embodiment the nucleic acid molecule is greater than 2 kb in length, the initial extension phase is between 5 minutes and 7 minutes, and subsequent extension phases are time varied phases. In another embodiment the nucleic acid molecule is greater than 3 kb, the initial extension phase is between 5 minutes and 7 minutes, and subsequence extension phases are progressively increased in time relative to the initial extension phase. The set of oligonucleotides can contain more than 100 oligonucleotides. One or more of the phases can be time varied phases. In a particular embodiment the extension phase is a time varied phase. The extension phase can be cumulatively extended by about 15 seconds per cycle, and the multiple cycles be at least 25 cycles. The nucleic acid molecule can have one or more AT rich sequences.

### Detailed Description of the Drawings

Figure 1A provides a graphical illustration of overlapping oligonucleotides, where oligonucleotides A and B, B and C, C and D, D and E, and E and F are overlapping oligonucleotides and are opposite adjacent oligonucleotides. Figure IB illustrates homology or overlapping sequences between double-stranded (ds) DNA fragments.
Figure 2 provides a graphical illustration of a set of gapped and ungapped oligonucleotides for Gene A.
Figure 3 provides an illustration of a gel showing the assembly of a 2.3 kb gene (mutS) from oligonucleotides according to the methods of the invention.
Figure 4 provides an illustration of a gel showing the assembly of a 3.7 kb gene from oligonucleotides according to the methods of the invention.
Figure 5 provides an illustration of a gel showing the assembly of AT rich DNA from oligonucleotides according to the methods of the invention.
Figure 6 provides an illustration of a gel showing the assembly of 7 DNA fragments to create a 7 kb molecule according to methods of the invention.
Figure 7 provides an illustration of a gel showing the assembly of the mutS gene from 86 oligonucleotides according to methods of the invention.

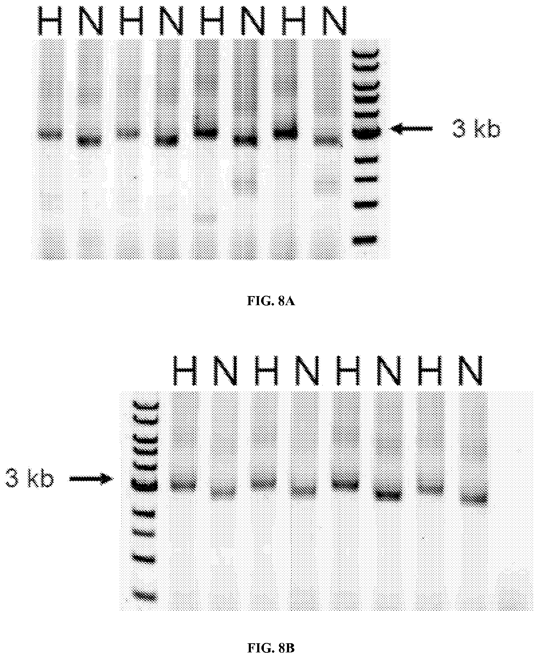

Figure 8A-B illustrates a 0.8% pre-cast agarose gel showing the assembly of nucleic acid constructs HA (H) and NA (N) from various influenza virus strains, each assembled from 96 pooled oligonucleotides in a system of the invention using the methods of the invention. Both Constructs HA and NA are of approximately 3 kb. Fig. 8A- Lane 1: A/Brisbane/10/2010(H1N1)_HA; Lane 2: A/Brisbane/10/2010(H1N1)_NA; Lane 3: X179A_TD(H1N1)_HA; Lane 4: X179A(H1N1)_NA; Lane 5: A/Victoria/361/2011_CDC/ E3(H3N2)_HA; Lane 6: A/Victoria/361/2011(H3N2)_NA; Lane 7: A/Brisbane/256/2011_P2/E3(H3N2)_HA; Lane 8: A/Brisbane/256/2011_P2/E3(H3N2)_NA; Standards lane. Fig 8B- Standards lane; Lane 1: B/Texas/06/2011_BX-45_HA; Lane 2: B/Texas/06/2011_BX-49_NA; Lane 3: B/New_Hampshire/1/2012_HA; Lane 4: B/New_Hampshire/1/2012_NA; Lane 5: B/Brisbane/60/08_HA; Lane 6: B/Brisbane/60/08_NA; Lane 7: B/Nevada/03/2011_v2_HA; Lane 8: B/Nevada/03/2011_v2_NA.

### Detailed Description of the Invention

By utilizing the methods of the present invention, the assembly of a desired nucleic acid molecule can be achieved in a single step. Thus according to the present invention both the time necessary and the cost of assembling hundreds of oligonucleotides is reduced. The invention thus facilitates goals related to the construction of diverse synthetic metabolic pathways, automated DNA assembly, and the robust engineering of large DNA fragments. The present invention is partially based on the discovery that the inclusion of a crowding agent in the assembly mixture offers beneficial properties in the assembly of nucleic acid molecules. Without wanting to be bound by any particular theory the present inventors believe that the inclusion of the crowding agent in the assembly mixture helps complementary oligonucleotides anneal to each other with higher specificity, thereby increasing the robustness of the nucleic acid assembly reaction.

The present invention takes advantage of the benefits of including a crowding agent in the assembly mixture, but also of the optimization of reaction temperature and reaction times for annealing and extension in nucleic acid assembly. By combining annealing and extension in a single step, oligonucleotide sequences in a set of oligonucleotides are allowed to anneal with specificity and serve as templates for nucleic acid extension. The methods allow for the assembly of longer nucleic acid fragments than has previously been possible and with lower cost. In some embodiments nucleic acid fragments longer than 1 kb and up to 7 kb and greater can be assembled and amplified. The present methods also allow for the assembly and/or amplification of nucleic acid molecules having high AT content or high GC content. Furthermore, the methods of the present invention allow for the elimination of nucleic acid assembly steps, and for the removal of certain enzymes to be included in the reaction mixture. The methods also allow for the assembly of significantly larger numbers of oligonucleotides than has previously been possible. Over a hundred single-stranded DNA oligonucleotides can be assembled from a mixture according to the methods and dozens of double-stranded DNA fragments can be assembled with the methods. The time and effort required to assemble nucleic acids having AT or GC rich sequences has been dramatically reduced with the present methods.

The invention is a single step or one step method for assembling a set of single-stranded overlapping oligonucleotides that comprise the length of a nucleic acid desired to be assembled or fragments thereof by contacting the set with a DNA polymerase, a mixture of dNTPs, and a crowding agent. By "single step" or "one step" is meant that once the reaction components are placed into a reaction vessel, the assembly and amplification of the desired nucleic acid molecule is achieved without needing to re-open the vessel. The methods of the invention therefore offer the opportunity to consolidate the assembly of a nucleic acid construct into a single step, thus combining a pre-assembly step and a PCR amplification and assembly step into a single reaction step. The single-stranded overlapping oligonucleotides can be assembled simultaneously.

### Methods

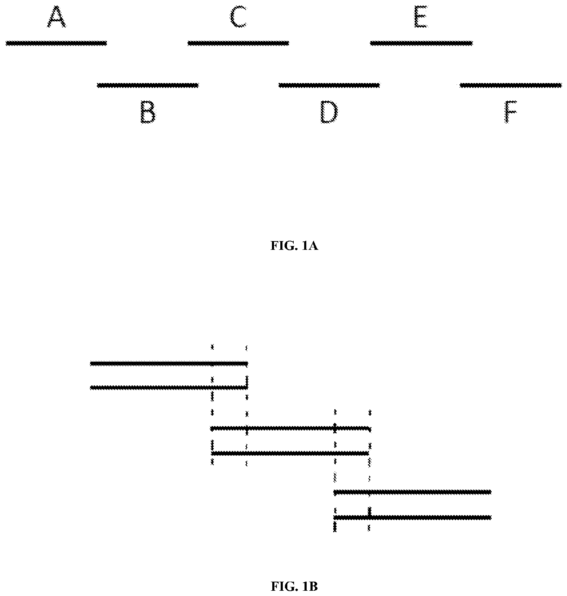

The invention provides methods for assembling nucleic acid molecules from a set of overlapping oligonucleotide fragments. A set of overlapping oligonucleotides means at least 2 overlapping oligonucleotides, but in other embodiments the set of oligonucleotides can contain any number of oligonucleotides as explained herein such as, for example, at least 50 or at least 70 or at least 100 or at least 150 oligonucleotides. The set of overlapping oligonucleotides contains oligonucleotides having sequences where at least a portion of the sequence of each oligonucleotide is complementary to and allows for annealing of the oligonucleotide to at least one other oligonucleotide (an anti-sense oligonucleotide) of the set. In various embodiments the oligonucleotides of the set can be from about 60 bases to about 70 bases in length. 60 base oligonucleotides can overlap the opposite adjacent (anti-sense) oligonucleotide by about 30 bp. A 70 base oligonucleotide can overlap its opposite adjacent (anti-sense) oligonucleotide by about 35 bp (see Fig. 1a). Each strand of the nucleic acid molecule to be assembled can be divided into suitable oligonucleotide fragments. In some embodiments this is done using appropriate software that will divide the sequence into a suitable number of overlapping fragments of suitable length as described herein, but in other embodiments is done simply by identifying suitable points of division. The set of overlapping oligonucleotides can be synthesized on a DNA or oligonucleotide synthesizer. In various embodiments the overlapping oligonucleotides of the set overlap the opposite adjacent (anti-sense) oligonucleotide by at least 10 nucleotides or by at least 20 nucleotides or at least 30 or at least 40 or more than 50 or more than 60 nucleotides. The set of oligonucleotides to be assembled can be pooled in a suitable vessel using a suitable buffer. The assembly mixture also contains a DNA polymerase, dNTPs, and a crowding agent, as described herein. The assembly mixture is then subjected to one or more cycles of nucleic acid assembly phases, which include one or more of an annealing phase, an extension phase, and a denaturation phase. While the phases can be performed in the recited order, in some embodiments they can also be performed in any order. The conditions of each phase are described herein. The result is assembly of the desired nucleic acid molecule from the set of overlapping oligonucleotides, which in one embodiment is done in a single step.

Overlapping (single-stranded) oligonucleotides are distinguished from overlapping (double-stranded) nucleic acid fragments. In some embodiments single-stranded oligonucleotides overlap their opposite adjacent (or anti-sense) oligonucleotide at complementary sequences, allowing the oligonucleotides to anneal to each other and the resulting gap can be filled in by a DNA polymerase, an embodiment of which is depicted in Fig 1a. When single-stranded oligonucleotides are assembled into a nucleic acid fragment, a plurality of the nucleic acid fragments can be assembled to arrive at a larger DNA construct. Nucleic acid fragments (double-stranded) can also have homology between the pieces, or overlapping sequences, for example at their respective ends as depicted in Fig. 1b. The overlapping sequences on the fragments can be utilized to assemble the fragments into a larger construct, for example by a "chew back" and repair method or other methods described herein. If it is desired to assemble a set of dsDNA fragments, these overlapping nucleic acid fragments will become overlapping oligonucleotides when subjected to the denaturation, annealing, and extension phases of the cycles of the methods. The methods are therefore useful for assembling a nucleic acid fragment from overlapping single-stranded oligonucleotides, an example of which is depicted in Fig. 1a, and are also useful for assembling a plurality of double-stranded nucleic acid fragments having overlapping sequences (or homology between the fragments), an example of which is depicted in Fig. 1b. In another embodiment the nucleic acid fragments have single-stranded overhangs, meaning that one or both of the strands of dsDNA extends beyond the double-stranded region of the dsDNA leaving a single-stranded overhang(s). The methods of the invention are also useful for assembling a mixture of single-stranded oligonucleotides and double-stranded DNA fragments in which the oligonucleotides can anneal to an overhang from the dsDNA, thus providing a manner of bridging or linking the single-stranded oligonucleotide and the dsDNA fragment together.

The present invention also provides optimized temperature and/or the time periods for annealing and extension phases in an assembly method. In one embodiment the invention combines annealing and extension in a single phase and thus allows complementary DNA sequences to anneal with specificity and serve as templates for PCR extension. Without being bound by any particular theory the present inventors believe that the addition of the crowding agent facilitates annealing of complementary oligonucleotides with even higher specificity, thereby increasing the robustness of the PCR reaction and the assembly of the nucleic acid.

In one embodiment the methods of the invention utilize a single step and a single temperature (i.e. isothermal) for PCR annealing and extension. In one embodiment the annealing and extension phases are combined and are performed isothermally, for example at a temperature of about 67 °C. In other embodiments at least the annealing phase occurs at a temperature of between 57 °C and 77 °C or between 50 °C and 77 °C, or the annealing and extension phases are combined and performed at a temperature of between 57 °C and 77 °C or between 50 °C and 77 °C. In different embodiments annealing and extension temperatures of about 50 °C ± 2 °C can be useful for the assembly of AT-rich DNA sequences. Annealing and extension temperatures of about 67 °C ± 2 °C can be useful for the assembly of GC-rich DNA sequences.

The method allows for the assembly of DNA molecules that are much longer than has been possible using previous methods. It was discovered unexpectedly that utilizing the method of the invention DNA molecules can be assembled that are about 4 times longer than has been previously been possible to assemble. The method can be used to assemble DNA fragments of about 1 kb in size, or greater than 1 kb. In other embodiments DNA molecules of greater than 2 kb or greater than 3 kb or greater than about 3.5 kb or greater than 4 kb or greater than 5 kb or greater than 6 kb or about 7 kb or greater than 7 kb can be assembled. In still more embodiments the methods allow for the assembly of DNA molecules of from 1-4 kb or from 1-5 kb or from 1-6 kb or from 1-7 kb or from 1-8 kb or from 1-9 kb or from 1-10 kb or from 2-5 kb or from 2-7 kb or from 2-8 kb or from 2-10 kb.

The methods of the invention are also useful for assembling a very large number of single-stranded (ss) oligonucleotides into a nucleic acid fragment. In various embodiments the methods can be used to assemble a set of more than 60 oligonucleotides or more than 80 or more than 100 or more than 120 or more than 140 or more than 160 or more than 180 or more than 200 oligonucleotides or from 60-200 or from 60-300 oligonucleotides into a double-stranded nucleic acid fragment.

### Crowding Agent

A crowding agent is an agent that causes, enhances, or facilitates molecular crowding. The crowding agent can bind water molecules. In one embodiment the crowding agent binds water molecules and does not bind to protein or nucleic acid molecules. Molecular crowding may occur by macromolecules reducing the volume of solvent available for other molecules in the solution. In the present invention, the crowding agent is polyethylene glycol (PEG). Any suitable PEG can be used in the compositions and methods of the invention. In various embodiments the PEG is PEG 12000 or PEG 10000 or PEG 8000 or PEG 4000 or PEG 2000 or PEG 1000. In other embodiments the PEG has a molecular weight greater than 4000, or greater than 6000 or greater than 8000 or greater than 10000 or greater than 12000. In other embodiments the PEG has a molecular weight of less than 4000, or less than 6000 or less than 8000 or less than 10000 or less than 12000. In still other embodiments the PEG is provided as a mixture of PEG molecules of differing sizes, e.g., any combination of the above listed PEG molecules. The PEG can be provided in the methods of the invention in any suitable concentration higher than 0.0188%, for example, 0.0375% or 0.075% or 0.3% or 0.45% or 0.6% or 0.75% or 1.0%, all w/v. In other embodiments of the methods of the invention the PEG is provided in a concentration of greater than 0.0375% or greater than 0.075% or greater than 0.3% or greater than 0.45% or greater than 0.6% or greater than 0.75% or greater 1.0%, all w/v. While PEG is used as crowding agent in the present invention, additional crowing agents can also be used in the methods such as, for example, albumins, Ficoll (e.g., Ficoll 70) and other high-mass, branched polysaccharides (e.g., dextran).

### Assembly Mixture

In one embodiment the assembly mixture is a combination of a set of overlapping oligonucleotides, a DNA polymerase, a mixture of dNTPs, and PEG. The assembly mixture may also contain additional components desirable for the method being performed. In a particular embodiment the PEG is PEG 8000. PEG of different molecular weights can be used or mixtures of PEG of different sizes can be used, such as a mixture of any of the sizes of PEG disclosed herein. The assembly mixture is normally present as a solution, but in some embodiments can be a dry mixture. In one embodiment the DNA polymerase and dNTPs are present in an amount sufficient to polymerize the overlapping oligonucleotides when they are annealed to produce a double-stranded DNA molecule when subjected to the method.

The method involves subjecting the assembly mixture to multiple cycles, i.e., one or more cycles. A cycle can include one or more of an annealing phase, an extension phase, and a denaturation phase. A cycle can also include more than one of any of the types of phases. Annealing involves the pairing by hydrogen bonds of an oligonucleotide to a complementary sequence on another oligonucleotide to form a double-stranded nucleic acid. Annealing can occur by any effective method, one method being the lowering of temperature of an assembly mixture to allow complementary sequences to anneal. Thus during the annealing phase the set of overlapping oligonucleotides anneal forming part or all of the length of the nucleic acid molecule to be assembled, leaving gaps where no nucleotides are present due to such regions being between the overlapping sequences. During the extension phase the set of oligonucleotides that have been annealed are acted upon by DNA polymerase, which will fill in the gaps left in areas where there were no complementary bases to anneal and form a base pair. The extension phase(s) will thus form a partially or complete double-stranded nucleic acid molecule. A ligase is optionally present in the assembly mixture, at suitable concentration for ligating annealed oligonucleotide strands. But in many embodiments a ligase is not necessary and the ligation will occur spontaneously. During the denaturation phase the double-stranded nucleic acid molecules are denatured into single-stranded oligonucleotides. Denaturation can be performed by heat denaturation. Through multiple cycles of one or more of such phases the desired nucleic acid molecule is assembled from the set of overlapping oligonucleotides. In one embodiment the methods are performed in a single step.

### Primers

In some embodiments the assembly mixture further comprises primers. In one embodiment the primers anneal to the end oligonucleotides on their 5' ends. The end oligonucleotides are those oligonucleotide fragments that form the 5' ends of the oligonucleotide strands that form the nucleic acid molecule to be assembled (in one example , oligonucleotides A and F in Fig. 1). As described herein, the nucleic acid molecule to be assembled is assembled from a set of overlapping oligonucleotide fragments. When the oligonucleotide fragments are annealed and when the DNA polymerase fills in the gaps, the desired nucleic acid molecule is assembled. The primers can be of any convenient size that functions in the methods. In various embodiments the primers can be about 10 nucleotides, or about 15 nucleotides, or about 18 nucleotides or about 20 nucleotides or about 25 nucleotides or about 30 nucleotides or about 35 nucleotides or longer or between 10 and 20 nucleotides or between 5 and 15 nucleotides or between 15 and 25 nucleotides or between 20 and 40 nucleotides or between 30 and 50 nucleotides or between 40 and 60 nucleotides or between 50 and 70 nucleotides. In one embodiment the primers are about 60 nucleotides.

In another embodiment no primers are present in the assembly mixture but the end oligonucleotides are present in a greater concentration than the other (non-end) oligonucleotides. The end oligonucleotides can be of any appropriate length as described herein, but in one embodiment the end oligonucleotides are about 60 nucleotides in length. The end oligonucleotides can also be present in different concentrations depending on the specific application, but in one embodiment are present at a concentration of about 500 nM. One example of end oligonucleotides are oligonucleotides A and F in Fig. 1. In another embodiment a mixture of primers and end oligonucleotides can be utilized. The non-end oligonucleotides are those oligonucleotides that are not the end oligonucleotides. Oligonucleotides

The oligonucleotides utilized in the invention can be of any suitable length. In various embodiments the oligonucleotides comprise 40-80 nucleotides, or 40-60 nucleotides, or 50-70 nucleotides, or about 60 nucleotides. But in other embodiments the oligonucleotides utilized in the invention can be of any length that functions in the methods. Additional examples include, but are not limited to, 20-40 nucleotides, 30-50 nucleotides, 40-60 nucleotides, or 50-70 nucleotides, or 60-80 nucleotides, or about 20 nucleotides, or about 30 nucleotides, or about 40 nucleotides, or about 50 nucleotides, or about 60 nucleotides, or about 70 nucleotides, or about 80 nucleotides, or more than 80 nucleotides.

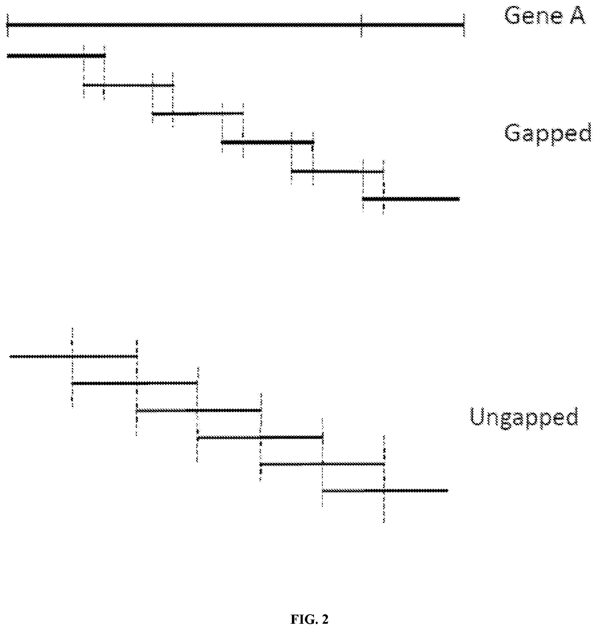

In one embodiment of the invention the oligonucleotides in the set of oligonucleotides are ungapped, i.e., utilize an ungapped alignment. Ungapped alignment means that when the oligonucleotides of the set are aligned, all nucleotides and/or sequences of the gene to be assembled are represented in at least two oligonucleotides of the set. In other embodiments a gapped set of oligonucleotides is used. An example of gapped and ungapped oligonucleotides is illustrated in Fig. 2.

In different embodiments the assembly mixture contains a set of at least 5 or at least 10 or at least 25 oligonucleotides, or at least 50 oligonucleotides, or at least 60 oligonucleotides, or at least 70 oligonucleotides, or at least 80 oligonucleotides, or at least 90 oligonucleotides, or at least 100 oligonucleotides, or at least 110 oligonucleotides, or at least 120 oligonucleotides, or at least 150 oligonucleotides. The set of oligonucleotides is assembled in a one-step reaction according to the invention. In other embodiments the assembly mixture contains between 50 and 100 oligonucleotides, or between 75 and 125 oligonucleotides, or between 100 and 150 oligonucleotides.

In different embodiments the oligonucleotides in the assembly mixture are present at a concentration of about 2.5 nM or between 2.0 nM and 3.0 nM. In embodiments using end primers the end primers can be present at a concentration of about 500 nM or from about 400 nM to about 600 nM. The person of ordinary skill in the art with reference to the present specification will realize that the specific concentration of oligonucleotides and/or end primers can be varied according to the reaction conditions selected.

### DNA Polymerase

The DNA polymerase used in the methods can be any suitable DNA polymerase. In particular embodiments a *Pyroccoccus*-like enzyme containing a processivity enhanced domain to permit increased processivity is also suitable. While any DNA polymerase may be used, a DNA polymerase delivering high accuracy and high processivity will be most effective. In some embodiments the DNA polymerase can also have 5' → 3' DNA polymerase activity or a 3' → 5' exonuclease activity. In one embodiment the DNA polymerase generates blunt ends in the amplification of products in DNA amplification reactions. Additional, non-limiting examples of DNA polymerases that can be used in the invention include DNA polymerase from *Pyrococcus furiosus,* which can be modified at one or more domains to provide greater activity and/or greater accuracy than the native enzyme. The modification can include a change in the nucleic acid sequence of the enzyme to provide for an enzyme with more advantageous properties in a DNA assembly procedure. The DNA polymerase can be heat stabile. The DNA polymerase can have all or only some of these properties, and the person of ordinary skill with resort to the present specification will realize which properties can be advantageously employed in a particular application of the methods and which reaction conditions and buffer components are appropriate for a particular DNA polymerase. One DNA polymerase that is suitable for the present methods is the commercially available PHUSION® High Fidelity DNA polymerase (Finnzymes, Oy, FI). Other DNA polymerases can also be suitable. In one embodiment a master mix can contain the DNA polymerase with MgCl₂ at suitable concentration (e.g., 1.5 mM), as well as a mixture of dNTPs at a suitable concentration (e.g., 200 uM of each dNTP at final reaction concentration) in 100% DMSO.

Any suitable reaction buffer can be used in the assembly reactions of the invention such as, for example, ISO buffer. Persons of ordinary skill in the art will realize additional buffers and conditions that are suitable for conducting the methods disclosed herein.

### Cycles and Phases

A cycle of the method is comprised of one or more phases, such as one or more of an annealing phase, one or more of an extension phase, and one or more of a denaturation phase. In one embodiment a cycle has an annealing phase, an extension phase, and a denaturation phase, but in some embodiments a cycle can have more than one of each type of phase. The method can utilize any convenient number of cycles necessary to perform the assembly. In various embodiments about 25 cycles or about 30 cycles or about 35 cycles are included in the methods. In other embodiments more than 20 cycles or more than 25 cycles or more than 30 cycles or more than 35 cycles are included in the method. In still more embodiments less than 25 cycles or less than 30 cycles or less than 35 cycles are included in the methods.

In some embodiments of the methods one or more of the phases can be a time varied phase. Any one of the phases can be a time varied phase, or all of the phases or any combination of phases can be time varied phases; thus there can be a time varied annealing phase and/or a time varied extension phase and/or a time varied denaturation phase. A time varied phase is a phase that is conducted for a period of time that varies or changes between cycles. A time varied phase (e.g., a time varied extension phase) of a cycle can be increased or decreased in time relative to the same phase of the prior cycle or relative to the first such phase of the cycle or relative to the phase of the first cycle of the method. For example, in one embodiment the extension phase of each cycle is a time varied phase. Thus, in one embodiment the first extension phase of a cycle is carried out at about 67 °C for about 6 min, and for one or more subsequent cycles the extension phase can be increased by about 15 seconds. In another embodiment the time varied phase can be increased or decreased in time relative to the second cycle of the method. The timewise extensions can be cumulative, thus if cycle 1 has an extension phase of 6 min, the cycle 2 extension phase can be about 6 minutes, 15 seconds (1:15), and cycle 3 can be about 6:30 (i.e., increase cumulatively by about 15 seconds per cycle), and so on. In various embodiments the timewise increase in a time varied phase can be an increase of about 5 seconds, or about 10 seconds, or about 15 seconds, or about 20 seconds or about 25 seconds or about 30 seconds or about 45 seconds or about 1 minute. Increases in any of the phases can be time varied and/or cumulative or non-cumulative from one cycle to the next. In some embodiments one or more annealing phases and/or denaturation phases are time varied, e.g., by extending the time of the phase for any of the periods described above, whether cumulatively or non-cumulatively. A combined annealing/extension phase can also be time varied as described herein. In different embodiments at least two cycles or at least three cycles can utilize a time varied phase.

The first extension phase of a cycle (or any extension phase of any cycle) can be simply an extension phase or can be a combined annealing/extension phase where both annealing and extension occur in the same phase. In different embodiments the first combined annealing/extension phase of a cycle (or a subsequent combined annealing/extension phase) can occur for a time period of at least 30 seconds/kilobase of nucleic acid being assembled, or at least 1 min/kb of nucleic acid being assembled, or at least 1.5 min/kb, or at least 2 min/kb, or at least 2.5 min/kb, or at least 3 min/kb of nucleic acid being assembled. In different embodiments the first extension phase or combined annealing/extension phase of a cycle can be for about 15 seconds, or about 30 seconds, or about 45 seconds, or about 1 min, or about 2 min, or about 3 min, or about 4 min, or about 5 min, or about 6 min, or about 7 min, or about 8 min, or about 9 min, or about 10 min. As described herein, subsequent extension phases or combined annealing/ extension phases can be time varied, and can be cumulatively increased or can be of the same time periods, as described herein.

During the denaturation phase nucleic acid molecules are denatured. In one embodiment heat denaturation is used. The heat denaturation can occur at a temperature of about 98 °C. But any temperature that serves to denature the nucleic acid molecules can be used, such as greater than or less than 70 °C or greater than or less than 80 °C or greater than or less than 90 °C or greater than 98 °C. The person of ordinary skill in the art with reference to the present disclosure will realize that the precise temperature of denaturation will depend on the precise composition and length of the nucleic acid molecule. The time of the denaturation phase can also vary depending on the precise composition and length of the nucleic acid. In some embodiments the denaturation phase can occur for 30 seconds. But in other embodiments the length of the denaturation phase can be greater or less than 30 seconds.

During the annealing phase the oligonucleotides of the set of oligonucleotides will find their complementary (anti-sense) sequences and anneal by forming double-stranded nucleic acid by hydrogen-bonding. The nucleic acid sequence will have gapped regions. During the annealing phase there can also be present with the set of oligonucleotides other assembly mixture components, which can include a DNA polymerase, a mixture of dNTPs, and a crowding agent (e.g., polyethylene glycol). Additional components can also be present such as a suitable buffer, buffer components, an optional ligase if desirable, as well as additional components.

During the extension phase the DNA polymerase polymerizes the dNTPs and fills in gaps left by the hybridization or annealing of the set of oligonucleotides (e.g. see Fig. 1). As described herein, in some embodiments the extension and annealing phases can be combined into a single phase. In various embodiments the temperature used in an annealing phase or combined annealing/extension phase in various embodiments can be about 65 °C, or about 66 °C, about 67 °C, or about 68 °C, or about 69 °C, or from about 65 to about 69 °C, or from about 66 °C to about 68 °C. The time period for a combined annealing/extension phase can vary depending on the length of nucleic acid sequence to be assembled. In various embodiments the combined annealing/extension phase can be about 1 min, or about 2 min, or about 3 min, or about 4 min, or about 5 min, or about 6 min, or about 7 min., or about 8 min, or about 9 min, or about 10 min. In various embodiments the time and temperature of the combined annealing/extension phase can be 67 °C for 1 min for a nucleic acid sequence of less than or equal to about 1 kb. In other embodiments a combined annealing/extension phase can be conducted at about 67 °C for about 6 min for a nucleic acid sequence of from about 2 kb to about 3 kb, or from about 2 kb to about 6 kb, or from about 3 kb to about 4 kb, or from about 3 kb to about 6 kb, or from about 2 kb to about 7 kb, or from about 2 kb to about 8 kb. In time varied formats the combined annealing/extension phase can be cumulatively increased by a suitable time period each cycle. For example, in some embodiments the time period for the combined annealing/extension phase can be cumulatively increased by about 15 seconds per cycle or by about 10 seconds/cycle or about 20 seconds per cycle. The number of cycles can vary depending on the particular application but in different embodiments about 30 cycles can be used, or about 25 cycles, or about 35 cycles, or about 40 cycles. Any suitable number of cycles can be used. Further examples include more than 20 cycles or more than 25 cycles or more than 30 cycles or more than 35 cycles.

Strands of DNA having AT rich sequences are often difficult to assemble. The methods of the present invention are able to assemble DNA molecules having AT rich sequences without difficulty. In different embodiments the AT rich sequences may have greater than 60% or greater than 65% or greater than 70% AT content. The methods can also assemble nucleic acids having high GC content, which are also often difficult to assemble due to inadequate strand separation and secondary structure formation. In embodiments using the combined annealing/extension phase for a nucleic acid sequence having an AT rich region, it is desirable to use a lower temperature. Thus, in one embodiment for assembling an AT rich nucleic acid sequence the temperature of the annealing/extension phase can be about 62 °C, or about 63 °C, or about 64 °C, or about 65 °C, or about 66 °C or about 67 °C. The time period for the combined phase in one embodiment is about 4 min. But in other embodiments the time period for the combined phase is about 3 min or about 5 min. In a particular embodiment the combined annealing/extension phase is carried out at 65 °C for about 4 min. The phases for AT rich sequences can be time varied as described herein.

### Kits

Outside the scope of the invention kits for performing a method of the invention, are disclosed. In one embodiment a kit contains a DNA polymerase, a mixture of dNTPs, and a crowding agent. The kit can also contain instructions for performing a method of the invention and/or information directing the user to a website or other resource that provides information about performing the methods. The DNA polymerase, dNTPs, and crowding agent contained in the kit can be provided in separate containers or in the same container. The DNA polymerase, dNTPs, and crowding agent can be any described herein.

### Example 1 - Assembly of PCR Products of Less than 1 kb

This example illustrates a comparison between a one step gene assembly method for PCR products of less than 1 kb in the presence versus the absence of a crowding agent (here PEG 8000).

Three genes were selected with lengths as follows: Gene 1: 32 oligonucleotides; Gene 2: 28 oligonucleotides; Gene 3: 30 oligonucleotides; Gene 4: 31 oligonucleotides. All oligonucleotides were from 60-70 bases in length. 60 base oligonucleotides had overhangs of 30 bases , and 70 base oligonucleotides had overhangs of 35 bases. For each gene, all oligonucleotides were pooled in a 50 ml tube by adding 5 ul of each oligo (100 uM stock). The volume was adjusted to 20 ml by adding 1xTE buffer, pH 8.0 to obtain a final oligonucleotide concentration of 25 nM/oligo.

ISO stock buffer was prepared with 0.75% PEG 8000. ISO buffer is used as a means to deliver PEG to the PCR reactions. It contains PEG-8000 in the desired amount, 600 mM Tris-HCl, pH 7.5, 40 mM MgCl₂, 40 mM DTT, 800 uM of each of the four dNTPs and 4 mM NAD. The stock was added to 2x PHUSION® Master Mix (Finnzymes Oy, FI) to obtain a final PEG concentration of 0.375% w/v. The Master Mix buffer contains DNA polymerase, nucleotides, and a reaction buffer containing MgCl₂. Persons of ordinary skill will realize that commercially-prepared mixes offer great convenience but other suitable buffers can be prepared.

To assemble oligonucleotides without PEG 8000 reactions were set up with 0.5 and 1 ul of the 25 nM/oligo mixture described above, and then there was added 2x PHUSION® Master Mix (Finnzymes Oy, FI), water, and A and B primers into the tubes. The total PCR volume was 20 ul.

To assemble oligonucleotides with PEG 8000, reactions were set up with 0.5 and 1 ul of the 25 nM/oligo mixture described above, and then there was added 2x PHUSION® Master Mix (Finnzymes Oy, FI) with 0.0375% PEG 8000, water, and A and B primers into the tubes. The total PCR volume was 20 ul.

**Table 1**

| Component | 20 ul rxn | Final Conc |
|---|---|---|
| Water | to 20 ul | |
| 2x Phusion | | |
| w/ or w/o | 10 ul | 1x |
| PEG 8000 | | |
| Primer A | 1 ul | 0.5 uM |
| Primer B | 1 ul | 0.5 uM |
| Template | 0.5-1 ul | |

The following assembly protocol was used:

| | |
|---|---|
| Step 1 | 98 °C for 30 sec. denaturation phase |
| Step 2 | 67 °C for 1 min combined first annealing/extension phase |
| Step 3 | increase time of annealing/extension phase 15 sec/cycle cumulative |
| | Repeating Steps 1-3 for a total of 30 cycles |

Total reaction time: about 2.5 hours

A 1.2% DNA gel was run with 5 ul of the above reactions. The gel showed that robust amplification of ungapped oligos can be achieved by combining the annealing and extension temperature at 67 °C. The oligonucleotide samples assembled in the presence of PEG provided a substantially more distinct gel band than those samples assembled in the absence of PEG.

### Example 2 - Assembly of a 2.3 kb Gene

This example illustrates a one step PCR assembly in the presence and absence of PEG 8000 for a 2.3 kb gene (mutS) from 86 ungapped oligonucleotides.

Oligos were pooled in a 50 ml tube by adding 5 ul of each oligo (100 uM stock). Volume was adjusted to 20 ml by adding 1xTE buffer, pH 8.0 to obtain a final oligo concentration of 25 nM/oligo. A 0.75% PEG 8000 stock was prepared in water. The stock was added to 2x PHUSION® Master Mix (Finnzymes Oy, FI) to obtain a final PEG concentration of 0.0054%, 0.0188%, 0.0375%, 0.075%, and 0.15%. All oligonucleotides used were from 60-70 bases in length. 60 base oligonucleotides had overhangs of 30 bases, and 70 bases oligonucleotides had overhangs of 35 bases.

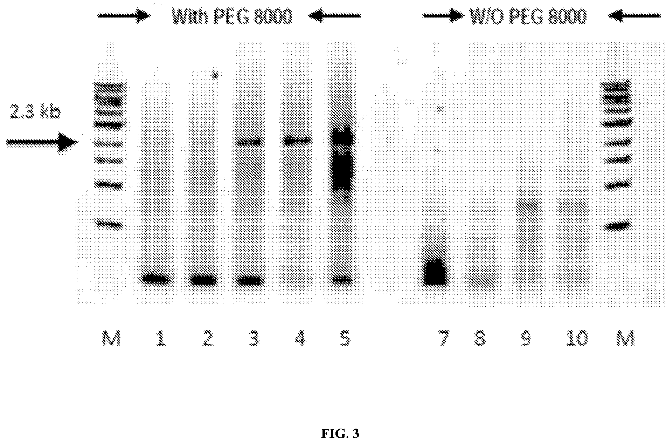

To assemble 86 oligos without PEG 8000, 0.5, 1.0, 1.5, and 2.5 ul (corresponding to Lanes 7-10 in Fig. 3, with Lane M a standards marker lane) of the above 25 nM oligo mixture was added to four PCR tubes and then Master Mix, water, and primers were added for a total PCR volume of 20 ul.

To assemble 86 oligos with PEG 8000, 1 ul of the 25 nM oligo mixture was added to five PCR tubes and then Master Mix with a final PEG 8000 concentration of 0.0054%, 0.0188%, 0.0375%, 0.075%, and 0.15% (corresponding to Lanes 1-5 in Fig. 3), water, and primers were added for a total PCR volume of 20 ul.

**Table 2**

| Component | 20 ul rxn | Final Conc |
|---|---|---|
| Water | to 20 ul | |
| 2x PHUSION® w/ or w/o PEG 8000 | 10 ul | 1x |
| Primer A (10 uM) | 1 ul | 0.5 uM |
| Primer B (10 uM) | 1 ul | 0.5 uM |
| Template | 0.5-3 ul | |

The following assembly protocol was used:

| | |
|---|---|
| Step 1 | 98 °C for 30 sec. |
| Step 2 | 67 °C for 6 min |
| Step 3 | increase time 15 sec/cycle |
| | Repeating Steps 1-3 for a total of 30 times |

Total reaction time: about 6 hours

A 1.2% DNA gel was run with 5 ul of the above reaction mixture. The gel is illustrated in Fig. 3. The gel shows that PCR conditions alone are not sufficient to assemble large DNA fragments and that PEG 8000 alone (without other ISO components) allows successful assembly. The example also illustrates that by providing for a combined first cycle annealing/extension phase of at least 2.5 min. per kb of nucleic acid being assembled, a 2.3 kb gene (mutS) was successfully assembled.

### Example 3 - Assembly of a 3.7 kb Gene From 124 Oligos

This example shows a one step PCR with and without PEG 8000 of a 3.7 kb gene (MetH) from 124 ungapped oligos. All oligonucleotides used were from 60-70 bases in length. 60 base oligonucleotides had overhangs of 30 bases, and 70 base oligonucleotides had overhangs of 35 bases.

Oligos were pooled in a 15 ml tube by adding 5 ul of each oligo (100 uM stock). Volume was adjusted to 10 ml by adding 1xTE buffer pH 8.0 to obtain a final oligo concentration of 50 nM/oligo. ISO stock buffer was prepared with 0.75% PEG 8000. Stock was added into 2x PHUSION® Master Mix (Finnzymes Oy, FI) to obtain final PEG 8000 concentrations of 0.0188%, 0.0375%, 0.075%, 0.3% and 0.45%.

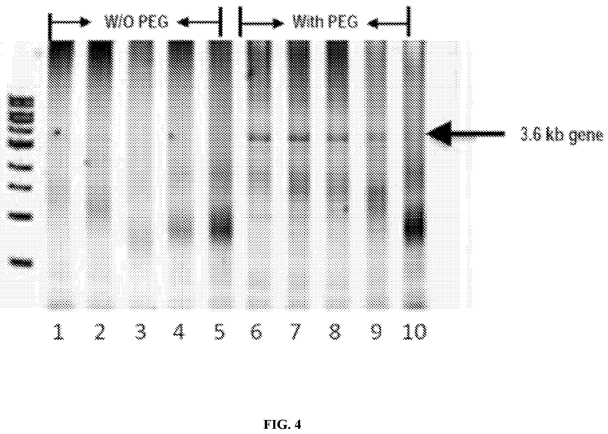

To assemble oligos without PEG 8000, 0.5, 1.0, 1.5, 2.0 and 3.0 ul (shown as Lanes 1-5 in Fig. 4, respectively) of the above 50 nM/oligo mixture was added to five PCR tubes and then Master Mix, water, and primers were added for a total PCR volume of 20 ul.

To assemble oligos with PEG 8000, 1 ul of the 50 nM oligo mixture was added to five PCR tubes and then Master Mix was added with 0.0188%, 0.0375%, 0.075%, 0.3%, and 0.45% of PEG 8000 (shown as Lanes 6-10 in Fig. 4), water, and primers for a total PCR volume of 20 ul.

**Table 3**

| Component | 20 ul rxn | Final Conc |
|---|---|---|
| Water | to 20 ul | |
| 2x PHUSION® w/ or w/o PEG 8000 | 10 ul | 1x |
| Primer A (10 uM) | 1 ul | 0.5 uM |
| Primer B (10 uM) | 1 ul | 0.5 uM |
| Template | 0.5-3 ul | |

The following assembly protocol was used:

| | |
|---|---|
| Step 1 | 98 °C for 30 sec. |
| Step 2 | 67 °C for 6 min |
| Step 3 | increase time 15 sec/cycle |
| | Repeating Steps 1-3 for a total of 35 times |

Total reaction time: about 7 hours

A 1.2% DNA gel was run with 3 ul of the above reactions and is illustrated as Fig. 4. The results showed that a 3.7 kb gene can be assembled from oligos according to the present invention.

### Example 4 - Assembly of an AT Rich Gene

This example illustrates a one step PCR assembly, with and without PEG 8000, of an AT rich 2.1 kb gene (dhaB1) from 70 ungapped oligonucleotides. Also shown is assembly of an AT rich 1.7 kb gene (dhaB) from 63 ungapped oligonucleotides with and without PEG 8000. All oligonucleotides used were from 60-70 bases in length. 60 base oligonucleotides had overhangs of 30 bases, and 70 bases oligonucleotides had overhangs of 35 bases.

Oligos were pooled in a 50 ml tube by adding 5 ul of each oligo (from 100 uM stock). Volume was adjusted to 20 ul by adding 1xTE buffer pH 8.0 to obtain a final oligo concentration of 25 nM/oligo.

ISO stock buffer was prepared with 0.75% PEG 8000 and has the components as described in Example 1. Stock was added into 2x PHUSION® Master Mix (Finnzymes Oy, FI) to obtain final PEG 8000 concentrations of 0.0375%.

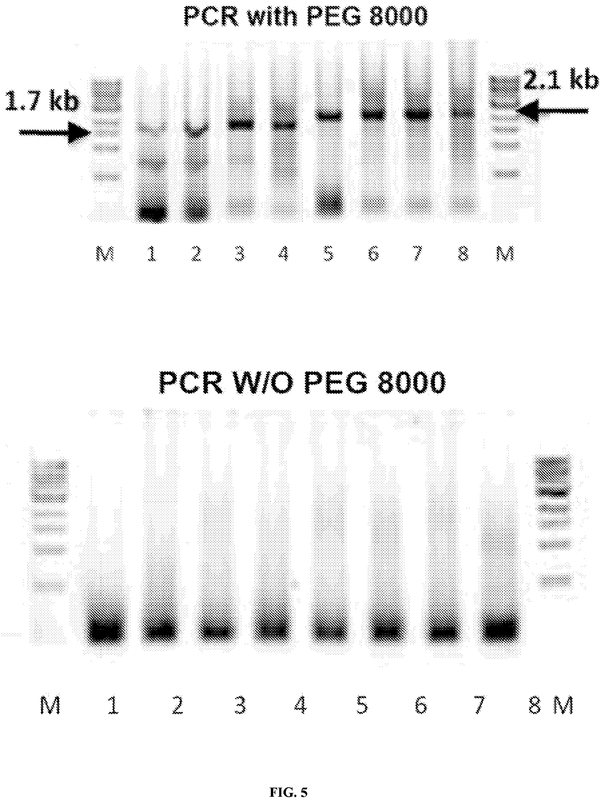

To assemble oligos without PEG 8000, 0.5, 1.0, 1.5, and 2.0 ul (shown as Lanes 1-4, respectively, for the 1.7 kb gene and Lanes 5-8, respectively for the 2.1 kb gene), in the "w/o PEG" gel of Fig. 5) of the above 20 nM oligo mixture was added to five PCR tubes and then Master Mix, water, and primers were added for a total PCR volume of 20 ul.

To assemble oligos with PEG 8000, 0.5, 1.0, 1.5, and 2.0 ul (shown as Lanes 1-4, respectively, for the 1.7 kb gene and Lanes 5-8, respectively, for the 2.1 kb gene in the "with PEG" gel of Fig. 5) of the 25 nM oligo mixture was added to five PCR tubes and then Master Mix was added with 0.0375% PEG 8000, water, and primers for a total PCR volume of 20 ul.

**Table 4**

| Component | 20 ul rxn | Final Conc |
|---|---|---|
| Water | to 20 ul | |
| 2x PHUSION® w/ or w/o PEG 8000 | 10 ul | 1x |
| Primer A (10 uM) | 1 ul | 0.5 uM |
| Primer B (10 uM) | 1 ul | 0.5 uM |
| Template | 0.5-2.5 ul | |

The following assembly protocol was used:

| | |
|---|---|
| Step 1 | 98 °C for 30 sec. |
| Step 2 | 67 °C for 4 min |
| Step 3 | increase time 15 sec/cycle |
| | Repeating Steps 1-3 for a total of 30 times |

Total reaction time: about 5 hours

A 1.2% DNA gel was run with 3 ul of the above reactions, and is illustrated as Fig. 5. The results show that assembly conditions alone are not sufficient to assemble large, AT rich (70%) DNA fragments and that the addition of a crowding agent (PEG 8000) facilitates successful assembly. It also shows that temperature can be lowered during the combined annealing/extension phase to facilitate assembly of AT rich DNA.

### Example 5 - Assembly of a 7 kb DNA Product From 7 Overlapping dsDNA Fragments

This example illustrates a one step PCR assembly, with or without PEG 8000, of 7 DNA fragments to create a 7 kb molecule. 7 DNA fragments (50 ng - 100 ng) having 30 bp homology (overlap) with each fragment were pooled. Fragment 1 of 250 bp; fragment 2 of 2,000 bp; fragment 3 of 1,000 bp; fragment 4 of 700 bp; fragment 5 of 1,500 bp; fragment 6 of 1,000 bp; and fragment 7 of 1,800 bp.

A stock of 0.75% PEG 8000 was prepared in water. The stock was added to 2x PHUSION® Master Mix (Finnzymes Oy, FI) to obtain final PEG 8000 concentrations of 0.0375% and 0.075%.

To assemble 7 DNA fragments without PEG 8000, 3.5 ul of the fragment mixture was added to a PCR tube and then Master Mix, water, and primers.

To assemble 7 DNA fragments with PEG 8000, 3.5 ul of the fragment mixture was added to two PCR tubes and then Master Mix was added with a final PEG concentration of 0.0375% and 0.075%, and then water, and primers A and B.

**Table 5**

| Component | 20 ul rxn | Final Conc |
|---|---|---|
| Water | to 20 ul | |
| 2x PHUSION® w/ or w/o PEG 8000 | 10 ul | 1x |
| Primer A (10 uM) | 1 ul | 0.5 uM |
| Primer B (10 uM) | 1 ul | 0.5 uM |
| Template | 3.5 ul | |

The following assembly protocol was used:

| | |
|---|---|
| Step 1 | 98 °C for 30 sec. |
| Step 2 | 67 °C for 6 min |
| Step 3 | increase time 15 sec/cycle |
| | Repeating Steps 1-3 for a total of 30 times |

Total reaction time: about 7 hours

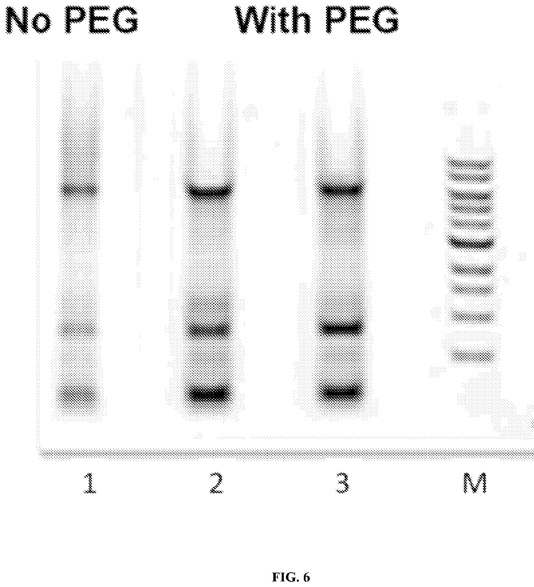

A 1.2% DNA gel was run with 3 ul of the above reactions, and is illustrated as Fig. 6, where Lane 1 contains no PEG, Lane 2 contains 0.0375% PEG, and Lane 3 contains 0.075% PEG, and Lane M is a marker lane. The results show robust amplification of overlapping DNA fragments is achieved by combining the annealing and extension temperature at 67 °C. PEG 8000 increases product.

### Example 6 - Assembly of mutS (86 Oligos)

This example illustrates a one step PCR assembly of mutS (86 oligos) comparing PEG and ISO. All oligonucleotides used were from 60-70 bases in length. 60 base oligonucleotides had overhangs of 30 bases, and 70 base oligonucleotides had overhangs of 35 bases.

Oligos were pooled in a 50 ml tube by adding 5 ul of each oligo (100 uM stock). Volume was adjusted to 20 ml by adding 1xTE buffer pH 8.0 to obtain a final oligo concentration of 25 nM/oligo.

A stock of 0.75% PEG 8000 was prepared in water. The stock was added to 2x PHUSION® Master Mix (Finnzymes Oy, FI) to obtain final PEG 8000 concentrations of 0.0188%, 0.028%, 0.0375% and 0.075%.

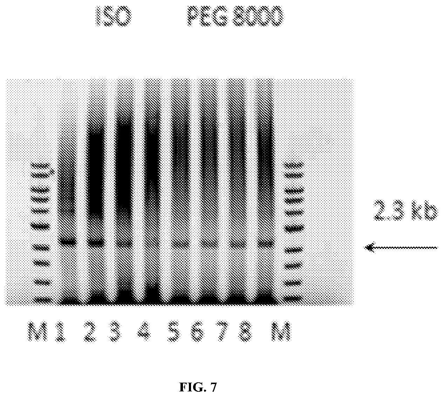

A stock of 0.75% PEG 8000 was prepared in ISO buffer, having the components as described in Example 1. The stock was added to 2x PHUSION® Master Mix (Finnzymes Oy, FI) to obtain final PEG 8000 concentrations of 0.0188%, 0.028%, 0.0375% and 0.075%, shown as Lanes 1-4 respectively in Fig. 7. Lanes 5-8 contained no PEG and M is a marker lane.

**Table 6**

| Component | 20 ul rxn | Final Conc |
|---|---|---|
| Water | to 20 ul | |
| 2x PHUSION® w/ or w/o PEG 8000 | 10 ul | 1x |
| Primer A (10 uM) | 1 ul | 0.5 uM |
| Primer B (10 uM) | 1 ul | 0.5 uM |
| Template | 0.5 to 3 ul | |

The following assembly protocol was used:

| | |
|---|---|
| Step 1 | 98 °C for 30 sec. |
| Step 2 | 67 °C for 6 min |
| Step 3 | increase time 15 sec/cycle |
| | Repeating Steps 1-3 for a total of 30 times |

Total reaction time: about 6 hours

A 1.2% DNA gel was run with 3 ul of the above reactions, and is illustrated as Fig. 7. The results show that the PEG is the component in ISO that improves PCR-mediated DNA assembly.

### Example 7 - Assembling Minimal Genome Sub-Assemblies

A minimal genome from a Mycoplasma was divided into 370 proposed fragments of approximately 1.4 kb each using the ARCHETYPE™ (Synthetic Genomics, Inc., San Diego, CA) software program. Each of these 370 fragments was in turn divided into about 44 (ungapped) oligonucleotides, each oligo approximately 70 nucleotides in length and containing an approximately 35 nucleotide overlap with the opposite adjacent oligo (i.e. approximately 35 nucleotides of repeat sequence on each adjacent double-stranded DNA). The flanking (or "end") oligonucleotides (e.g. oligo # 1 and oligo # 44) for the fragments contained 30 nucleotides of a sequence common to all 370 fragments (for use in PCR amplification) and 8 bases of sequence containing a restriction site (e.g. NotI) for release of the insert from the vector. Each of the 370 fragments also contained an overlapping sequence of 60 nucleotides to the adjacent double-stranded nucleic acid fragment such that they could be recombined for a subsequent stage of assembly.

Once oligonucleotides comprising each of the 370 sub-assemblies were pooled, they were diluted to a concentration of 200 nM per oligo. The assembly reaction, which both assembles the oligonucleotides and amplifies the resulting product in a single step, is shown below:
50 ul 2X Q5 polymerase mix (NEB)
0.8 ul 5% PEG-8000
0.5 ul primer 1 [pUC19 Insert F]
0.5 ul primer 2 [pUC19 Insert R]
1.25 ul 200nM oligo pool above
46.95 ul water

We found that, for these high A/T content DNA samples, it beneficial to anneal/extend at 60 °C or lower.

The following cycling conditions were used and worked across a wide range of DNA sequences:
Cycling conditions:
   1. 98 °C 1 min
   2. 98 °C 10s
   3. 57 °C 30 seconds
Slow cool (0.1C/s) to 40C
   4. 40 °C 30 seconds
   3. 57 °C 6 min
Increase 15 sec every cycle
   4. Go to step 2 29 additional times
   5. 72C 5 minutes
   6. 10 °C -----

The assemblies can then be subjected to further stages of assembly, where the 1.4 kb constructs were assembled into 74 constructs of 6.7 kb each. These 6.7 kb constructs were then assembled into 8 constructs of 50-75 kb each, which were then assembled into a minimal Mycoplasma genome of 483 kb.

### Example 8 - Synthesis of a Functional HA and NA DNA Molecules and Protein Moieties

This example illustrates the automated assembly of DNA constructs of HA and NA genes from an oligonucleotide pool. Influenza viruses are made of a viral envelope containing glycoproteins wrapped around a central core. The central core contains the viral RNA genome and other viral proteins that package and protect the RNA. The influenza genome typically contains eight pieces of RNA with each containing one or two genes encoding viral proteins. In the case of influenza A, the genome contains 11 genes on eight pieces of RNA, encoding for 11 proteins, including hemagglutinin (HA) and neuraminidase (NA). Other proteins include nucleoprotein (NP), M1, M2, NS 1, NS2, PA, PB1, PB1-F2 and PB2.

Hemagglutinin (HA) and neuraminidase (NA) are glycoproteins present on the outside of the viral particles. These glycoproteins have key functions in the life cycle of the virus, including assisting in binding to host cells and reproduction of viral particles. The assembled virus containing these proteins is therefore useful in the production of a vaccine.

### Oligonucleotide Synthesis and Assembly

A pool of 96 oligonucleotides representing the sequence of DNA constructs of the HA and NA genes were provided to an assembly unit of the invention. The HA and NA constructs were approximately 3 kb in length and were assembled from 96 oligonucleotides in the method. The first and last oligonucleotides contained primer binding domains for PCR amplification and NotI restriction sites to release the primer binding domains following amplification and expose overlapping regions for DNA assembly, if necessary to assemble larger fragments.

The assembly unit utilized a BIOMEK® NXP, Span-8 laboratory automation workstation (Beckman Instruments Inc., Fullerton, CA) with integrated thermal-cycling capabilities.

The assembly unit was programmed to perform several different steps in the process namely, 1) PRC1 amplification to amplify oligonucleotides; 2) an error correction step on the PCR1 amplified oligonucleotides; 3) a PCR2 step to amplify the corrected oligonucleotides; 4) a PCR product purification step to provide pure amplified oligonucleotides; 5) an assembly step to assemble the oligonucleotide products into a gene. Each process can be performed in a distinct reaction zone of the reaction container (which is a 96 well plate), and the reaction zone can be one or more columns on the 96 well plate. Assembly reaction is at 50 °C for 30-60 minutes and the reaction is temperature shifted and held at 10 °C thereafter.

### 1st PCR and error correction

For each assembled product PCR reactions were performed in automated fashion:
25 ul 2X PHUSION® Hot-Start Master Mix (Thermo Fisher Scientific Oy, Oy, F1)
2 ul 1% PEG 8000
0.25 ul Terminal Primer 1 (100 uM)
0.25 ul Terminal Primer 2 (100 uM)
20 ul MBG water
2.5 ul of the oligo pool above was transferred at 50 nM as template to a reaction zone of the reaction container containing PCR master mix (or combine subsequently).
2. Thermal-cycle occurred using the following parameters:
   98 °C for 1 min
   30X (98 °C 30 sec, 65 C 6 minutes and extending that by 15 sec/cycle
      72 °C for 5 min
      10 °C forever

### PCR Purification

PCR product was purified using the AMPURE® XP technology (Agencourt, Bioscience Corp. Beverly, MA)

### GIBSON ASSEMBLY® (Synthetic Genomics, San Diego, CA) to combine sub-assemblies into HA and NA genes Within Plasmid Vectors.

Nucleic acid constructs of approximately 3 kb were produced. The electrophoretic gels are shown in Fig. 8. These genes already include promoter regions (pol I and pol II) for expression following transfection into mammalian cells.

## Claims

1. A method for assembling a nucleic acid molecule in a single step from a set of overlapping oligonucleotides, the method comprising:
(a) contacting a set of overlapping oligonucleotides with
a DNA polymerase;
a mixture of dNTPs; and
polyethylene glycol at a concentration of greater than 0.0188%: in a reaction vessel;
to form an assembly mixture;
(b) subjecting the assembly mixture to multiple cycles, each cycle comprising an annealing phase, an extension phase, and a denaturation phase,
(c) thereby assembling the nucleic acid molecule from a set of overlapping oligonucleotides in a single step.

2. The method of claim 1 wherein the set of oligonucleotides comprises end oligonucleotides and non-end oligonucleotides, and the end oligonucleotides are provided in the assembly mixture at a higher concentration than the non-end oligonucleotides.

3. The method of any of claims 1-2 wherein at least one annealing phase occurs at a temperature of between 50 °C and 77 °C, or wherein the extension phase of a cycle is increased in time relative to the extension phase of the previous cycle, or wherein the DNA polymerase is a modified DNA polymerase from *Pyrococcus furiosus,* or wherein the set of oligonucleotides is assembled into a gene, or wherein the annealing phase occurs at 67 °C, or wherein the annealing phase and the extension phase occur at 67 °C.

4. The method of any of claims 1-3 wherein the polyethylene glycol is PEG 8000.

5. The method of any of claims 1-4 wherein the concentration of PEG is 0.025% or greater.

6. The method of any of claims 1-5 wherein the concentration of PEG is 0.375% or greater.

7. The method of any of claims 1-6 wherein the nucleic acid molecule is greater than 1 kb in length, optionally wherein the nucleic acid molecule is greater than 2 kb in length, or is greater than 3 kb in length.

8. The method of any of claims 1-7 wherein the set of overlapping oligonucleotides comprises at least 5 oligonucleotides.

9. The method of any of claims 1-8 wherein the set of overlapping oligonucleotides comprises at least 60 oligonucleotides.

10. The method of any of claims 1-9 wherein the set of overlapping oligonucleotides comprises at least 75 oligonucleotides.

11. The method of any of claims 1-10 wherein the nucleic acid molecule assembled is greater than 2 kb, the initial extension phase is between 5 minutes and 7 minutes, and subsequent extension phases are time varied phases.

12. The method of any of claims 1-11 wherein the nucleic acid molecule assembled is greater than 3 kb, the initial extension phase is between 5 minutes and 7 minutes, and subsequence extension phases are progressively increased in time relative to the initial extension phase, optionally wherein the set of overlapping nucleotides comprises more than 100 oligonucleotides.

13. The method of any of claims 1-12 wherein one or more phases are time varied phases.

14. The method of any of claims 1-13 wherein the extension phase is a time varied phase, optionally wherein the extension phase is cumulatively extended by about 15 seconds per cycle.

15. The method of any of claims 1-14 wherein the multiple cycles comprise at least 25 cycles, or wherein the nucleic acid molecule assembled comprises one or more AT rich sequences.

## Patentansprüche

1. Verfahren zum Zusammensetzen eines Nukleinsäuremoleküls in einem einzigen Schritt aus einem Satz überlappender Oligonukleotide, wobei das Verfahren Folgendes umfasst:
(a) In-Kontakt-Bringen eines Satzes überlappender Oligonukleotide mit einer DNA-Polymerase;
einem Gemisch von dNTPs; und
Polyethylenglycol mit einer Konzentration von mehr als 0,0188%: in einem Reaktionsgefäß;
um ein Zusammensetzungsgemisch auszubilden;
(b) Unterwerfen des Zusammensetzungsgemisches unter multiple Zyklen, wobei jeder Zyklus eine Temperphase, eine Extensionsphase und eine Denaturierungsphase umfasst,
(c) dadurch Zusammensetzen des Nukleinsäuremoleküls aus einem Satz überlappender Oligonukleotide in einem einzigen Schritt.

2. Verfahren nach Anspruch 1, wobei der Satz von Oligonukleotiden End-Oligonukleotide und Nicht-End-Oligonukleotide umfasst, und, wobei die End-Oligonukleotide in dem Zusammensetzungsgemisch mit einer höheren Konzentration als die Nicht-End-Oligonukleotide vorgesehen sind.

3. Verfahren nach einem der Ansprüche 1 bis 2, wobei zumindest die Temperphase bei einer Temperatur von zwischen 50°C und 77°C auftritt, oder wobei die Extensionsphase eines Zyklus relativ zu der Extensionsphase des vorigen Zyklus zeitlich verlängert wird, oder wobei die DNA-Polymerase eine modifizierte DNA-Polymerase von *Pyrococcus furiosus* ist, oder wobei der Satz von Oligonukleotiden zu einem Gen zusammengesetzt ist, oder wobei die Temperphase bei 67°C auftritt, oder wobei die Temperphase und die Extensionsphase bei 67°C auftreten.

4. Verfahren nach einem der Ansprüche 1 bis 3, wobei das Polyethylenglycol PEG 8000 ist.

5. Verfahren nach einem der Ansprüche 1 bis 4, wobei die Konzentration von PEG 0,025% oder höher ist.

6. Verfahren nach einem der Ansprüche 1 bis 5, wobei die Konzentration von PEG 0,037% oder höher ist.

7. Verfahren nach einem der Ansprüche 1 bis 6, wobei das Nukleinsäuremolekül größer als 1 kb in der Länge ist, optional wobei das Nukleinsäuremolekül größer als 2 kb in der Länge ist oder größer als 3 kb in der Länge ist.

8. Verfahren nach einem der Ansprüche 1 bis 7, wobei der Satz überlappender Oligonukleotide mindestens 5 Oligonukleotide umfasst.

9. Verfahren nach einem der Ansprüche 1 bis 8, wobei der Satz überlappender Oligonukleotide mindestens 60 Oligonukleotide umfasst.

10. Verfahren nach einem der Ansprüche 1 bis 9, wobei der Satz überlappender Oligonukleotide mindestens 75 Oligonukleotide umfasst.

11. Verfahren nach einem der Ansprüche 1 bis 10, wobei das zusammengesetzte Nukleinsäuremolekül größer als 2 kb ist, die anfängliche Extensionsphase zwischen 5 Minuten und 7 Minuten liegt und folgende Extensionsphasen zeitvariierte Phasen sind.

12. Verfahren nach einem der Ansprüche 1 bis 6, wobei das zusammengesetzte Nukleinsäuremolekül größer als 3 kb ist, die anfängliche Extensionsphase zwischen 5 Minuten und 7 Minuten liegt und folgende Extensionsphasen in der Zeit relativ zu der anfänglichen Extensionsphase schrittweise verlängert werden, optional wobei der Satz überlappender Nukleotide mehr als 100 Oligonukleotide umfasst.

13. Verfahren nach einem der Ansprüche 1 bis 12, wobei eine oder mehrere Phasen zeitvariierte Phasen sind.

14. Verfahren nach einem der Ansprüche 1 bis 13, wobei die Extensionsphase eine zeitvariierte Phase ist, optional wobei die Extensionsphase um je 15 Sekunden pro Zyklus kumulativ ausgedehnt wird.

15. Verfahren nach einem der Ansprüche 1 bis 14, wobei die multiplen Zyklen mindestens 25 Zyklen umfassen, oder wobei das zusammengesetzte Nukleinsäuremolekül eine oder mehrere AT-reiche Sequenzen umfasst.

## Revendications

1. Procédé d'assemblage d'une molécule d'acide nucléique en une seule étape à partir d'un ensemble d'oligonucléotides à chevauchement, le procédé comprenant :
(a) la mise en contact d'un ensemble d'oligonucléotides à chevauchement avec
une ADN polymérase ;
un mélange de dNTP ; et
du polyéthylène glycol à une concentration supérieure à 0,0188 % dans un récipient de réaction ;
pour former un mélange d'assemblage ;
(b) la soumission du mélange d'assemblage à de multiples cycles, chaque cycle comprenant une phase de recuit, une phase d'extension et une phrase de dénaturation,
(c) ce qui permet d'assembler la molécule d'acide nucléique à partir d'un ensemble d'oligonucléotides à chevauchement en une seule étape.

2. Procédé selon la revendication 1, dans lequel l'ensemble d'oligonucléotides comprend des oligonucléotides d'extrémité et des oligonucléotides hors extrémité, et où les oligonucléotides d'extrémité sont présents dans le mélange d'assemblage à une concentration supérieure à celle des oligonucléotide hors extrémité.

3. Procédé selon l'une quelconque des revendications 1 ou 2, dans lequel l'au moins une phase de recuit à une température comprise entre 50 et 77 °C ou dans lequel la phase d'extension d'un cycle augmente au cours du temps par rapport à la phase d'extension du cycle précédent, ou dans lequel l'ADN polymérase est une ADN polymérase modifiée à partir de *Pyrococcus furiosus,* ou dans lequel l'ensemble d'oligonucléotides est assemblé en un gène, ou dans lequel la phase de recuit a lieu à 67 °C ou dans lequel la phase de recuit et la phase d'extension ont lieu à 67 °C.

4. Procédé selon l'une quelconque des revendications 1 à 3, dans lequel le polyéthylène glycol est le PEG 8000.

5. Procédé selon l'une quelconque des revendications 1 à 4, dans lequel la concentration de PEG est supérieure ou égale à 0,025 %.

6. Procédé selon l'une quelconque des revendications 1 à 5, dans lequel la concentration de PEG est supérieure ou égale à 0,375 %.

7. Procédé selon l'une quelconque des revendications 1 à 6, dans lequel la molécule d'acide nucléique a une longueur supérieure à 1 kb, la molécule d'acide nucléique ayant éventuellement une longueur supérieure à 2 kb ou une longueur supérieure à 3 kb.

8. Procédé selon l'une quelconque des revendications 1 à 7, dans lequel l'ensemble d'oligonucléotides à chevauchement comprend au moins 5 oligonucléotides.

9. Procédé selon l'une quelconque des revendications 1 à 8, dans lequel l'ensemble d'oligonucléotides à chevauchement comprend au moins 60 oligonucléotides.

10. Procédé selon l'une quelconque des revendications 1 à 9, dans lequel l'ensemble d'oligonucléotides à chevauchement comprend au moins 75 oligonucléotides.

11. Procédé selon l'une quelconque des revendications 1 à 10, dans lequel la molécule d'acide nucléique assemblé dépasse 2 kb, la phase initiale d'extension étant comprise entre 5 et 7 minutes, et les phases suivantes d'extension étant des phases variables au cours du temps.

12. Procédé selon l'une quelconque des revendications 1 à 11, dans lequel la molécule d'acide nucléique assemblée dépasse 3 kb, la phase initiale d'extension est comprise entre 5 et 7 minutes et les phases suivantes d'extension augmente progressivement au cours du temps par rapport à la phase initiale d'extension, dans lequel l'ensemble de nucléotides à chevauchement comprend éventuellement plus de 100 oligonucléotides.

13. Procédé selon l'une quelconque des revendications 1 à 12, dans lequel au moins une phase est une phase variable au cours du temps.

14. Procédé selon l'une quelconque des revendications 1 à 13, dans lequel la phase d'extension est une phase variable au cours du temps, la phase d'extension étant éventuellement cumulativement étendue d'environ 15 s par cycle.

15. Procédé selon l'une quelconque des revendications 1 à 14, dans lequel les cycles multiples comprennent au moins 25 cycles, ou dans lequel la molécule d'acide assemblée comprend au moins une séquence riche en A-T.
